# EUROPEAN PATENT SPECIFICATION

(11) **EP 2 976 114 B1**
(45) Date of publication and mention of the grant of the patent: **05.09.2018**
(21) Application number: 14722733.4
(22) Date of filing: 21.03.2014
(51) Int. Cl.: A61M 5/14, A61M 5/165, A61M 5/168, A61M 5/19, A61M 5/28, A61M 5/31, A61M 5/315

(54) **DUAL CHAMBER SYRINGE WITH PERMEABLE DIAPHRAGM FOR FORMING EMULSION**
ZWEIKAMMERSPRITZE MIT PERMEABLER MEMBRAN ZUR BILDUNG EINER EMULSION
SERINGUE À DOUBLE COMPARTIMENTS AVEC MEMBRANE PERMÉABLE POUR FORMER ÉMULSION

(30) Priority: 22.03.2013 IT MO20130075
(43) Date of publication of application: 27.01.2016
(73) Proprietor: Neuron Guard S.r.l., 41124 Modena (IT)
(72) Inventor: GIULIANI, Enrico, 41124 Modena (mo) (IT)
(74) Representative: Bergamini, Silvio
(86) International application number: PCT/IB2014/060032
(87) International publication number: WO 2014/147594

(56) References cited:
- EP-A1- 0 059 694
- WO-A1-2006/025902
- WO-A2-2008/008845
- DE-A1- 10 110 126
- US-A- 5 330 426
- US-A- 5 685 846
- US-A1- 2009 299 328

## Description

### Field of the Invention

The invention relates to an administration device, which can be generally used for administering drugs and drug mixtures to patients, particularly for administering metered doses of halogenated anesthetic agents to patients who suffered hypoxic or anoxic neuronal injury.

### Background art

It has been known for some time that patients who suffered hypoxic or anoxic brain injury after surgery or trauma may be treated with sedative drugs to decrease neuronal metabolism and, as a result, uncontrolled expansion of tissue damage, thereby limiting the occurrence of invalidating residual disabilities.

Particularly, US2004/0127578 discloses a method for cardioprotection and neuroprotection by intravenous administration of halogenated volatile anesthetics.

In the preferred embodiment of the invention, the amount of the formulation administered to the patient is a sub-anesthetic.

The formulation may be administered prior to, concurrently with, or after the ischemic event.

Nevertheless, this prior art suffers from certain drawbacks.

A first drawback is that the administered formulations comprise lipid solutions that have preservation problems, as they can be aggressed by contaminating pathogens.

Another drawback is that these lipid solutions have storage problems and a limited range of administration temperatures.

A further drawback is that these lipid solutions may induce allergenicity in particularly predisposed patients.

Yet another drawback is that continuous intravenous administration of a lipid solution throughout the treatment period may expose the body of the patient to a detrimental overload, and cause liver and lung alterations that would increase cardiovascular hazard.

A further drawback is that patients that have undergone an ischemic event are generally required to be treated in a hospital, whereby the suffering patient must be first moved from the place in which the event occurred, which will cause the loss of precious time to implement the required therapies and limit propagation of neuronal injury, which progressively necrotizes cells as time passes, due to neuronal hyperactivity induced thereby. Document WO 2006025902 discloses a syringe having two chambers separated by a permeable membrane. Each chamber contains a substance of a medicament. Actuating a pusher, both substances are mixed.

### Disclosure of the invention

The invention has the object to improve the prior art.

Another object of the invention is to provide an administration device that can be generally used for administering drugs and drug mixtures to patients, particularly for administering metered doses of halogenated anesthetic agents to patients who suffered hypoxic or anoxic neuronal injury, and allows effective treatment of patients in the place where an ischemic event has occurred.

A further object of the invention is to provide an administration device that allows administration of metered doses of anesthetic agents to patients either in quick, "bolus" form, i.e. with short administration times, typically five minutes or less, or as a continuous infusion, with the doses of anesthetic agents being allowed to be continuously metered according to the particular conditions of the patients. The invention provides an administration device as defined by the features of claim 1.

The invention achieves the following advantages:
- prompt administration of metered doses of halogenated anesthetic agents to patients that have suffered an ischemic event, to decrease neuronal activity in the brain and limit the occurrence of irreversible tissue injury;
- continuous metering of administered doses according to the conditions of the patients being treated;
- continuous parenteral administration of halogenated anesthetic agents.

### Brief description of the drawings

Further features and advantages of the invention will be more readily apparent upon reading of the detailed description of preferred non-exclusive embodiments of an administration device, which is shown as a non-limiting example in the annexed drawings, in which:
FIG. 1 is a perspective view of a first possible embodiment of an administration device of the invention, at the start of administration;
FIGS. 3 to 5 are schematic longitudinal sectional views of the administering device of Figure 1, during administration;
FIG. 6 is a detail view of a reference member that is adapted to be removably mounted to the administration device;
FIG. 7 is a cross-sectional view of the reference member of Figure 6, as taken along a plane VI - VI;

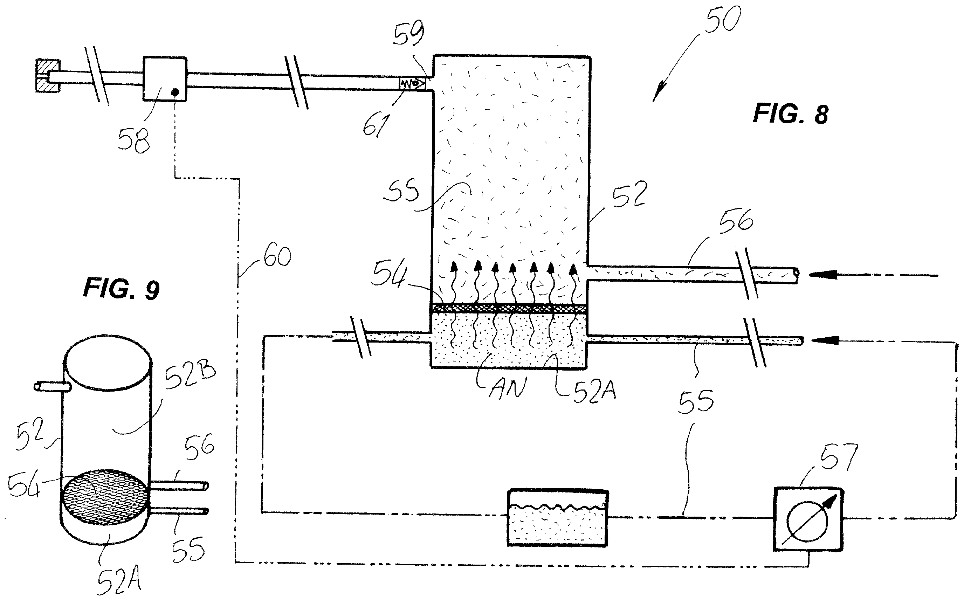

FIG. 8 is a schematic view of a second possible embodiment of the administration device of the invention;
FIG. 9 is a smaller-scale perspective view of the administration device of Figure 8.

### Detailed description of a preferred embodiment

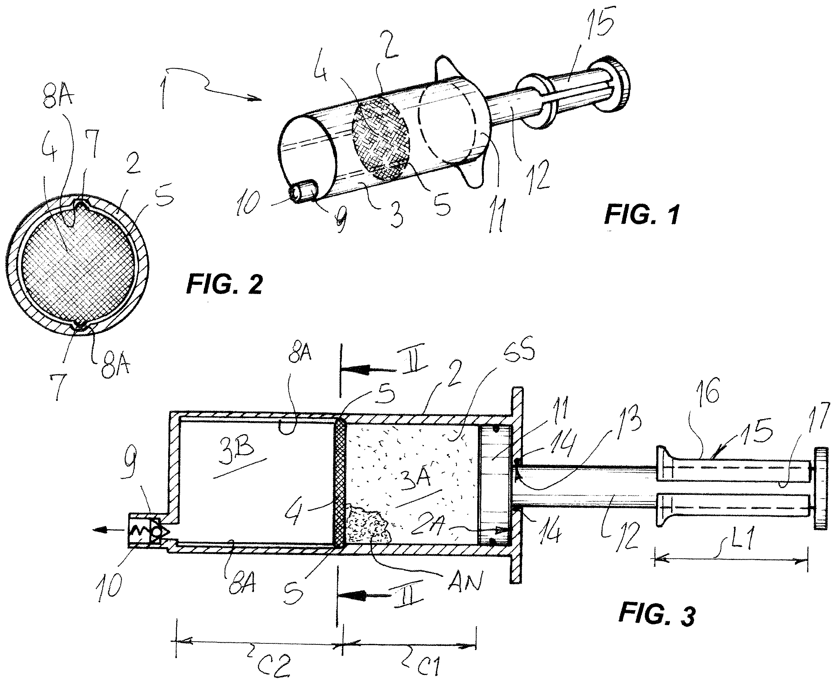

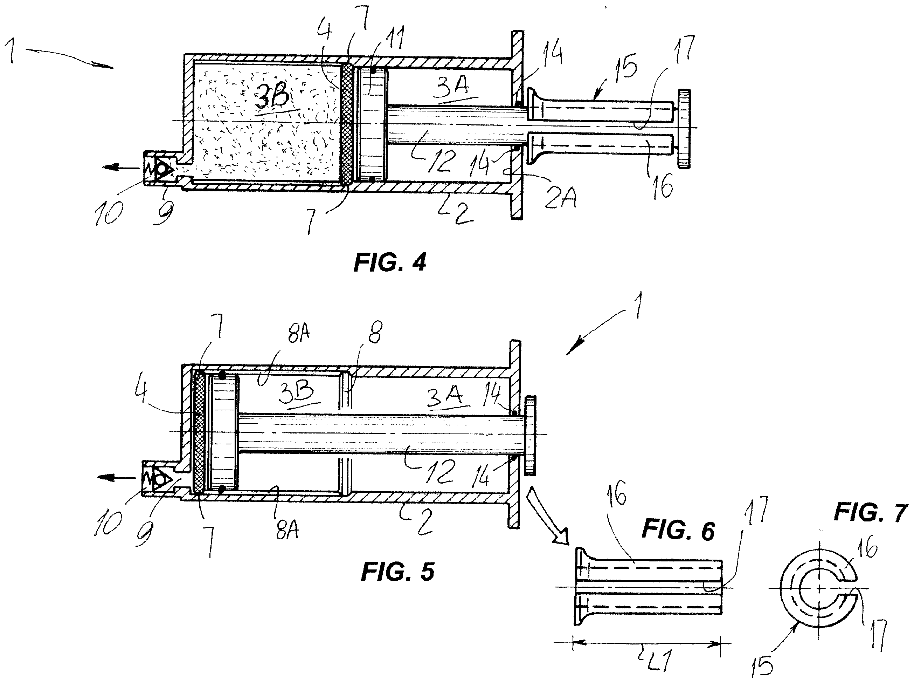

Referring now to Figures 1 to 5, numeral 1 generally designates an administration device according to a first embodiment, particularly but without limitation adapted for "bolus" administration for quick administration and, as needed, in the place where an ischemic event occurs.

In this embodiment, the device 1 is provided in the form of a syringe which typically comprises a container body 2 with a containing chamber 3 defined therein.

A microporous and hence permeable diaphragm 4 is transversely mounted in this chamber 3 using support means.

The diaphragm 4 comprises a stretching and support frame 5 which circumscribes the inner microporous part and is removably mounted in the containing chamber 3 using coupling and support means.

The latter comprise a raised peripheral rib 7, which extends from the frame 5 and is designed to be interlockingly received in a corresponding concave annular seat 8 formed in the containing chamber 3, and a pair of parallel longitudinal guides 8A arranged in the emulsification half-chamber 3B.

In practice, the diaphragm 4 divides the containing chamber into two half-chambers, i.e. a first loading half-chamber 3A and a contiguous second emulsification half-chamber 3B.

Furthermore, the second half-chamber 3B has an outlet 9 for the emulsified substances to be administered to a patient, which is equipped with a non-return valve 10 that prevents backflow of the substances into the second emulsification chamber 3B.

In a pre-administration condition, the first loading chamber 3A slidably and sealingly houses a plunger 11 having a shaft that coaxially extends out of the container chamber 3 through an opening 13 formed in a proximal wall 2A of the body 2 and having seals 14.

A removable reference member 15 is temporarily mounted to the stem 12, which has the purpose of pushing the plunger 11 into the container chamber toward the outlet 9, and its length, referenced "L1" in the figures is substantially equal to the length of a first stroke "C1" that the plunger 11 has to run to move from the proximal wall 2A to contact with the diaphragm 4.

The reference member 15 is actually a sleeve 16 with a longitudinal slit extending throughout its length "L1", which allows it to elastically open apart to a sufficient extent as to allow it to be fitted onto the shaft 12 or be removed therefrom.

In the first loading chamber 3A two substances to be administered to a patient that has suffered an ischemic event are loaded, for decreasing neuronal injury in the brain.

More in detail, these two substances comprise a halogenated and volatile anesthetic agent, schematically referenced "AN", and a solvent solution "SS", which are separate from each other, and they are originally in two different, non-emulsifiable states.

The diaphragm 4 may be formed either as a filter or as a membrane, but in both cases it is of microporous nature, for both substances to pass therethrough when the plunger 11 is pushed by the shaft 12 actuated by a medical operator, as better explained hereinafter.

In the second embodiment of the administration device, which is particularly suitable for sustained administration, instead of "bolus" administration, the administration device of the invention is referenced 50 and still comprises a body 52 defining therein a first loading half-chamber 52A and a contiguous second emulsification half-chamber 52B.

The two half-chambers are also divided by a microporous diaphragm 54, which is very similar to the diaphragm 4 as used in the previously described embodiment of the administration device 1.

A feeding circuit 55 is connected to the first half-chamber 52A for feeding the halogenated and volatile anesthetic agent "AN", and a feeding line 56 is connected to the second half-chamber 52B for feeding the solvent solution "SS".

An adjustable-flow pump 57 is also mounted to the circuit 55, to maintain the anesthetic agent "AN" in circulation, and create a pressure in the first half-chamber 52A, which is higher than the pressure in the second half-chamber 52B, to push the anesthetic agent "AN" through the diaphragm 54.

Like in the previously described embodiment, the second half-chamber 52B has an outlet 59 through which the emulsified substances may reach a patient that is designed to be treated thereby.

The outlet 59 has both the non-return valve 61 and a detection device 58 for detecting the concentration of the anesthetic agent "AN" in the solvent solution "SS", mounted thereto.

This detection device 58 is typically known to the skilled person and may be provided, for instance, in the form of an optical sensor, which is connected to the pump 57 via a connection line 60, to automatically control flow according to current detections, as constantly compared with the desired and pre-settable concentration values.

The operation of the administration device in the first embodiment of the invention is as follows: a dose of the anesthetic agent "AN" and a dose of the solvent solution "SS" are loaded into the first loading half-chamber 3A in which they remain in separate form, due to their different, not spontaneously emulsifiable states.

The medical operator fits the reference member 15 onto the shaft 12, if this has not been done at the start, and opens it apart enough to straddle the shaft and coaxially surround it in loose fashion.

For administration of the doses of the two substances in the first half-chamber 3A, the operator pushes the shaft 12 and causes the plunger 11 to contact the diaphragm 4 through a first stroke "C1".

During this first stroke "C1", both the dose of the anesthetic agent "AN" and the dose of the solvent solution "SS" pass through the micropores of the diaphragm 4 and the anesthetic agent "AN", which is typically of lipid nature, breaks up into microparticles that move into the second emulsification half-chamber 3B with the solvent solution "SS", and emulsify therewith due to their crushed state.

In this configuration, the reference member 15 rests upon the outer face of the proximal wall 2A to inform the medical operator that both substances have been pushed into the emulsification chamber 3B.

Therefore, the medical operator will remove the reference member 15 and push the shaft 12 again.

The plunger will push the diaphragm 4 and remove it from the concave annular seat 8 to progressively guide it toward the outlet 9 and also push both emulsified substances therethrough toward the receiving patient.

The one-way valve 10 prevents any backflow of both emulsified substances during administration.

In the second embodiment of the administration device 50, the pushing action that forces the anesthetic agent "AN" to pass through the diaphragm 54 that breaks it up into micro-particles is generated by the pressure created by the pump 57 that is mounted to the feeding circuit 55.

More in detail, the pump 57 keeps the anesthetic agent "AN" circulating in the circuit 55, while the solvent solution "SS" is fed into the second half-chamber 52B.

The overpressure created in the first half-chamber 52A generates a thrust that forces the anesthetic agent "AN" to pass through the microporous diaphragm 54 and break up into micro-particles that flow into the second half-chamber 52B and emulsify with the solvent solution "SS".

When both substances are emulsified, they are pushed toward the outlet 59, through which they reach the receiving patient.

As they pass through the outlet 58, the detection device 58 detects the concentration of the micro-particles of anesthetic agent "AN" in the solvent solution "SS" and compares it with a desired value that has been preset by the medical operator.

If the detected value does not match the preset value, then the detection device will control the pump 57 through the connection line 60 to change its flow for the concentration of the anesthetic agent "AN" to match the preset value.

The invention has been found to fulfill the intended objects.

The invention so conceived is susceptible to changes and variants within the inventive concept.

Furthermore, all the details may be replaced by other technically equivalent parts.

In practice, any material, shape and size may be used as needed, without departure from the scope as defined by the following claims.

## Claims

1. An emulsifier device (1; 50) comprising:
- A container body (2; 52) which defines in the inside a containing chamber (3) of at least two substances (AN, SS) to be administrated in at least two different states which cannot be emulsified spontaneously;
- A fragmentation permeable diaphragm (4; 54) which divides said containing chamber (3) into a first loading semi-chamber (3A; 52A) of substances (AN, SS) to be emulsified and an adjacent second mixing semi-chamber (3B; 52B) of said at least two substances to be emulsified;
- Pushing means (11, 12), **characterized in that** the pushing means pushes together said at least two substances to be administrated from said first semi-chamber (3A; 52A) into said second semi-chamber (3B; 52B) both passing through said permeable diaphragm (4; 54) whereby one substance breaks up into micro-particles that move into the second mixing semi-chamber (3b; 52B) wherein they emulsify and to push said at least two substances (AN, SS) already emulsified from said second semi-chamber (3B; 52B) to an administration addressee;
- one outlet (9; 59) from said second semi-chamber (3B; 52B) toward said addressee, wherein at least one of said two substances is a solvent solution and **in that** both said at least two substances (AN, SS) are loaded into said first loading semi-chamber (3A; 52A) wherein they remain in separated due to their not spontaneously emulsifiable states.

2. A device as claimed in claim 1, wherein said outlet (9; 59) comprises concentration sensing means (58) of at least one of said substances to be emulsified.

3. A device as claimed in claim 1 or 2, wherein said permeable diaphragm (4; 54) is chosen between a semi-permeable membrane or microporous filter.

4. A device as claimed in anyone of preceding claims, wherein said diaphragm (4; 54) is movably supported in said containing chamber (3) by supporting means (8).

5. A device as claimed in anyone of preceding claims, wherein said permeable diaphragm (4; 54) is peripherally equipped with a stretching and supporting frame (5), movably coupled with said support means (8) by coupling means (7).

6. A device as claimed in anyone of claims 1, 4, 5, wherein said support means comprise:
- A concave annular seat (8) transversally obtained in the inside of said containing chamber (3) and wherein said coupling means (7) are designed to be engaged in a removable way;
- A couple of parallel grooves (8A) extending from said annular seat (8) toward said outlet (9; 59) and wherein said coupling means (7) are slidingly received.

7. A device as claimed in claim 1, wherein said pushing means comprise a plunger (11) slidingly housed in said containing chamber (3) and movable between said first semi-chamber (3A; 52A) and second semi-chamber (3B; 52B) along a first movement stroke (C1) and a subsequent second movement stroke (C2), said plunger having a push stem (12) sealingly prolonging out of said containing chamber (3).

8. A device as claimed in claim 7, wherein said push stem (12) has a length at least equal to the sum of said first movement stroke (C1) and second movement stroke (C2).

9. A device as claimed in claim 7, wherein a reference element (15) having a length equal to said first movement stroke (C1) is placed on said push stem (12).

10. A device as claimed in claim 1, wherein said pushing means comprise a feeding circuit (55) of at least one of said substances to be emulsified and flowing into said first semi-chamber (3A; 52A).

11. A device as claimed in claim 10, wherein said circuit comprises a pressurized circuit (55) equipped with pumping means (57), designed to create a pressure inside said first semi-chamber (3A; 52A) greater than the pressure inside said second semi-chamber (3B; 52B).

## Patentansprüche

1. Emulgatorvorrichtung (1; 50), umfassend:
- einen Behälterkörper (2; 52), der im Inneren eine Aufnahmekammer (3) von mindestens zwei Substanzen (AN, SS) definiert, die in mindestens zwei verschiedenen, nicht spontan emulgierbaren Zuständen zu verabreichen sind;
- eine durchlässige Membran-Unterteilung (4; 54), die die Aufnahmekammer (3) in eine erste Ladehalbkammer (3A; 52A) von zu emulgierenden Substanzen (AN, SS) und eine benachbarte zweite Mischhalbkammer (3B; 52B) der mindestens zwei zu emulgierenden Substanzen unterteilt;
- Schubmittel (11 12), **dadurch gekennzeichnet, dass** das Schubmittel die mindestens zwei Substanzen, die von der ersten Halbkammer (3A; 52A) in die zweite Halbkammer (3B; 52B) zu verabreichen sind, zusammenschiebt, wobei beide durch die durchlässige Membran (4; 54) hindurchgehen, wodurch eine Substanz in Mikropartikel zerfällt, die sich in die zweite Misch-Halbkammer (3b; 52B) bewegen, wobei sie emulgieren und die mindestens zwei Substanzen (AN, SS), die bereits von der zweiten Halbkammer (3B; 52B) emulgiert sind, zu einem Verabreichungsempfänger schiebt;
- einen Ausgang (9; 59) aus der zweiten Halbkammer (3B; 52B) in Richtung des Empfängers, wobei mindestens eine der zwei Substanzen eine Lösungsmittel-Lösung ist und dass beide der mindestens zwei Substanzen (AN, SS) in die erste Ladehalbkammer (3A; 52A) geladen werden, wobei sie aufgrund ihrer nicht spontan emulgierbaren Zustände getrennt bleiben.

2. Vorrichtung nach Anspruch 1, wobei der Auslass (9; 59) Konzentrationserfassungsmittel (58) von mindestens einer der zu emulgierenden Substanzen umfasst.

3. Vorrichtung nach Anspruch 1 oder 2, wobei die durchlässige Membran (4; 54) zwischen einer semipermeablen Membran oder einem mikroporösen Filter ausgewählt ist.

4. Vorrichtung nach einem der vorstehenden Ansprüche, wobei die Membran (4; 54) in der Aufnahmekammer (3) durch Stützmittel (8) beweglich gelagert ist.

5. Vorrichtung nach einem der vorstehenden Ansprüche, wobei die durchlässige Membran (4; 54) peripher mit einem Streck- und Stützrahmen (5) ausgestattet ist, der durch Kupplungsmittel (7) beweglich mit den Stützmitteln (8) gekoppelt ist.

6. Vorrichtung nach einem der Ansprüche 1, 4, 5, wobei die Stützmittel umfassen:
- einen konkaven ringförmigen Sitz (8), der quer in der Innenseite der Aufnahmekammer (3) erhalten wird, und wobei die Kupplungsmittel (7) so ausgebildet sind, dass sie in einer entfernbaren Weise in Eingriff gebracht werden können;
- ein paar parallele Nuten (8A), die sich von dem ringförmigen Sitz (8) zu dem Ausgang (9; 59) erstrecken und wobei die Kupplungsmittel (7) gleitend aufgenommen werden.

7. Vorrichtung nach Anspruch 1, wobei die Schubmittel einen Kolben (11) umfassen, der gleitend in der Aufnahmekammer (3) untergebracht ist und zwischen der ersten Halbkammer (3A; 52A) und der zweiten Halbkammer (3B; 52B) entlang eines ersten Bewegungshubs (C1) und eines nachfolgenden zweiten Bewegungshubs (C2) bewegbar ist, wobei der Kolben eine Schubstange (12) aufweist, die sich abgedichtet aus der Aufnahmekammer (3) heraus erstreckt.

8. Vorrichtung nach Anspruch 7, wobei die Schubstange (12) eine Länge aufweist, die mindestens gleich der Summe von dem ersten Bewegungshub (C1) und dem zweiten Bewegungshub (C2) ist.

9. Vorrichtung nach Anspruch 7, wobei ein Bezugselement (15) mit einer Länge gleich dem ersten Bewegungshub (C1) auf der Schubstange (12) angeordnet ist.

10. Vorrichtung nach Anspruch 1, wobei die Schubmittel einen Zuführkreislauf (55) von mindestens einer der zu emulgierenden Substanzen umfassen und in die erste Halbkammer (3A; 52A) fließen.

11. Vorrichtung nach Anspruch 10, wobei die Schaltung einen Druckkreislauf (55) umfasst, der mit einer Pumpeinrichtung (57) ausgestattet ist, die dazu ausgelegt ist, einen Druck innerhalb der ersten Halbkammer (3A; 52A) zu erzeugen, der größer ist als der Druck innerhalb der zweiten Halbkammer (3B; 52B).

## Revendications

1. Dispositif d'émulsion (1; 50) comprenant:
- un corps de récipient (2; 52) qui définit intérieurement une chambre de stockage (3) d'au moins deux substances (AN, SS) à administrer dans au moins deux états différents qui ne peuvent pas être émulsifiées spontanément;
- un diaphragme de fragmentation perméable (4; 54) qui divise ladite chambre
de stockage (3) en une première demi-chambre de chargement (3A; 52A) de substances (AN, SS) à émulsifier et en une seconde demi-chambre de mélange adjacente (3B 52B) desdites au moins deux substances à émulsifier;
- des moyens de poussée (11, 12), **caractérisé en ce que** les moyens de poussée poussent ensemble lesdites au moins deux substances à administrer à partir de ladite première demi-chambre (3A; 52A) dans ladite seconde demi-chambre (3B; 52B) les deux passant à travers ledit diaphragme perméable (4, 54) par lequel une substance se décompose en microparticules qui se déplacent dans la seconde demi-chambre (3b; 52B) dans laquelle elles s'émulsifient et poussent lesdites au moins deux substances (AN, SS) déjà émulsifées à partir de ladite seconde demi-chambre (3B; 52B) vers un destinataire de l'administration;
- une sortie (9; 59) de ladite seconde demi-chambre (3B; 52B) vers ledit destinataire, dans lequel au moins une des deux substances est une solution de solvent et **en ce que** lesdites au moins deux substances (AN, SS) sont chargées dans ladite première demi-chambre (3A; 52A) dans laquelle elles restent sous une forme séparée, en raison de leurs états non spontanément émulsifiables.

2. Dispositif tel que revendiqué dans la revendication 1, dans lequel ladite sortie (9; 59) comprend des moyens de détection de concentration (58) d'au moins l'une desdites substances à émulsifier.

3. Dispositif tel que revendiqué dans la revendication 1 ou 2, dans lequel ledit diaphragme perméable (4; 54) est choisi entre une membrane semi-perméable ou un filtre microporeux.

4. Dispositif tel que revendiqué dans l'une quelconque des revendications précédentes, dans lequel ledit diaphragme (4; 54) est monté de manière mobile dans ladite chambre de stockage (3) par des moyens de support (8).

5. Dispositif tel que revendiqué dans l'une quelconque des revendications précédentes, dans lequel ledit diaphragme perméable (4; 54) est équipé périphériquement d'un cadre tendeur et de support (5), couplé de manière mobile auxdits moyens de support (8) par des moyens de couplage (7).

6. Dispositif tel que revendiqué dans l'une quelconque des revendications 1, 4, 5, dans lequel lesdits moyens de support comprennent:
- un siège annulaire concave (8) obtenu transversalement à l'intérieur de ladite chambre de stockage (3) et dans lequel lesdits moyens de couplage (7) sont conçus pour être engagés de manière amovible;
- un couple de rainures parallèles (8A) s'étendant depuis ledit siège annulaire (8) vers ladite sortie (9; 59) et dans lequel lesdits moyens de couplage (7) sont reçus de manière coulissante.

7. Dispositif tel que revendiqué dans la revendication 1, dans lequel lesdits moyens de poussée comprennent un plongeur (11) logé de manière coulissante dans ladite chambre de stockage (3) et mobile entre ladite première demi-chambre (3A; 52A) et la seconde demi-chambre (3B; 52B) le long d'une première course de déplacement (C1) et d'une seconde course de déplacement subséquente (C2), ledit plongeur ayant une tige de poussée (12) s'étendant de manière étanche hors de ladite chambre de stockage (3).

8. Dispositif tel que revendiqué dans la revendication 7, dans lequel ladite tige de poussée (12) a une longueur au moins égale à la somme desdites première course de déplacement (C1) et seconde course de déplacement (C2).

9. Dispositif tel que revendiqué dans la revendication 7, dans lequel un élément de référence (15) ayant une longueur égale à ladite première course de mouvement (C1) est placé sur ladite tige de poussée (12).

10. Dispositif tel que revendiqué dans la revendication 1, dans lequel lesdits moyens de poussée comprennent un circuit d'alimentation (55) d'au moins l'une desdites substances à émulsifier et à écouler dans ladite première demi-chambre (3A; 52A).

11. Dispositif tel que revendiqué dans la revendication 10, dans lequel ledit circuit comprend un circuit sous pression (55) équipé de moyens de pompage (57), conçus pour créer une pression à l'intérieur de ladite première demi-chambre (3A; 52A) supérieure à la pression à l'intérieur de ladite seconde demi-chambre (3B; 52B).
